(19) Europäisches Patentamt
European Patent Office
Office européen des brevets

(11) Veröffentlichungsnummer: **0 003 758**
**A1**

# (12) EUROPÄISCHE PATENTANMELDUNG

(21) Anmeldenummer: **79100273.6**

(22) Anmeldetag: **31.01.79**

(51) Int. Cl.²: **C 07 D 235/26**
**A 61 K 31/415**

(30) Priorität: **09.02.78 CH 1450/78**
**23.05.78 CH 5594/78**

(43) Veröffentlichungstag der Anmeldung:
**05.09.79 Patentblatt 79/18**

(84) Benannte Vertragsstaaten:
**BE CH DE FR GB IT LU NL SE**

(71) Anmelder: **CIBA-GEIGY AG**
**Patentabteilung Postfach**
**CH-4002 Basel(CH)**

(72) Erfinder: **Jaeggi, Knut A., Dr.**
**General Guisan-Strasse 44**
**CH-4054 Basel(CH)**

(72) Erfinder: **Ostermayer, Franz, Dr.**
**Am Hang 5**
**CH-4125 Riehen(CH)**

(72) Erfinder: **Schröter, Herbert, Dr.**
**Arisdörferstrasse 16**
**CH-4414 Füllinsdorf(CH)**

(54) **Verätherte Hydroxy-benzodiheterocyclen und ihre Säureadditionssalze, Verfahren zu deren Herstellung und diese enthaltende pharmazeutische Präparate.**

(57) Die vorliegende Erfindung betrifft verätherte Hydroxy-benzodiheterocyclen der Formel

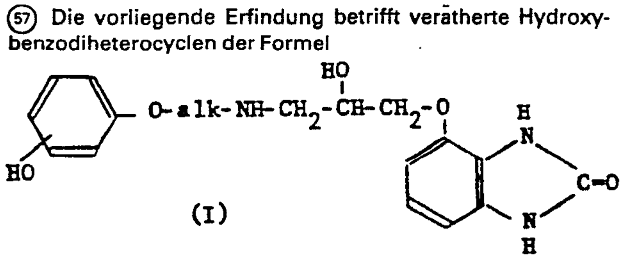

(I)

worin alk vicinales Alkylen mit 2 - 3 Kohlenstoffatomen bedeutet, sowie Säureadditionssalze davon und Verfahren zur Herstellung dieser Verbindungen sowie pharmazeutische Präparate enthaltend Verbindungen der Formel I oder pharmazeutisch annehmbare Säureadditionssalze davon. Die erfindungsgemässen neuen Stoffe besitzen wertvolle pharmakologische Eigenschaften, insbesondere zeigen sie starke und lang andauernde, cardioselektive Betarezeptorenblokkierende Wirkungen sowie auch Alpharezeptorenblockierende Wirkungen und können z.B. bei der Behandlung von Herzrythmusstörungen und coronaren Herzerkrankungen, sowie als blutdrucksenkende Mittel verwendet werden.

EP 0 003 758 A1

Croydon Printing Company Ltd.

Case 4-10290/3/4/=

Verätherte Hydroxy-benzodiheterocyclen und ihre Säureadditionssalze, Verfahren zu deren Herstellung und diese enthaltende pharmazeutische Präparate

Die vorliegende Erfindung betrifft verätherte Hydroxybenzodiheterocyclen, insbesondere Verbindungen der Formel

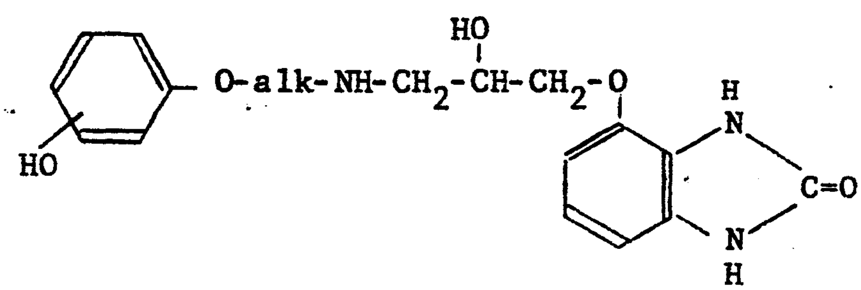

(I)

worin alk vicinales Alkylen mit 2 - 3 Kohlenstoffatomen bedeutet, sowie Säureadditionssalze davon und Verfahren zur Herstellung dieser Verbindungen sowie pharmazeutische Präparate enthaltend Verbindungen der Formel I oder pharmazeutisch annehmbare Säureadditionssalze davon, und die Verwendung dieser Verbindungen, vorzugsweise in Form von pharmazeutischen Präparaten.

In den Verbindungen der Formel I ist vicinales Alkylen alk 1- oder 2-Methyläthylen und vor allem Aethylen.

Säureadditionssalze von Verbindungen der Formel I sind insbesondere pharmazeutisch annehmbare, nicht-toxische Säureadditionssalze mit geeigneten anorganischen Säuren, wie Chlorwasserstoffsäure, Bromwasserstoffsäure, Schwefelsäure oder Phosphorsäure, oder mit geeigneten organischen aliphatischen, cycloaliphatischen, aromatischen, araliphatischen oder heterocyclischen Carbon- oder Sulfonsäuren, wie Ameisensäure, Essigsäure, Propionsäure, Bernsteinsäure, Glykolsäure, Milchsäure, Aepfelsäure, Weinsäure, Zitronensäure, Maleinsäure, Fumarsäure, Brenztraubensäure, Benzoesäure, Anthranilsäure, 4-Hydroxybenzoesäure, Salicylsäure, Phenylessigsäure, Embonsäure, Methansulfonsäure, Aethansulfonsäure, Hydroxyäthansulfonsäure, Aethylensulfonsäure, 4-Chlorbenzolsulfonsäure, Toluolsulfonsäure, Naphthalinsulfonsäure, Sulfanilsäure oder Cyclohexylaminsulfonsäure, oder weiteren sauren organischen Stoffen, wie Ascorbinsäure.

Infolge der engen Beziehungen zwischen den neuen Verbindungen in freier Form und in Form ihrer Säureadditionssalze sind unter den freien Verbindungen und unter den Salzen sinn- und zweckgemäss gegebenenfalls auch die entsprechenden Salze bzw. freien Verbindungen zu verstehen. Die neuen Verbindungen können in Form von Racematen oder von Antipoden vorliegen.

Die neuen Verbindungen weisen wertvolle pharmakologische Eigenschaften, insbesondere starke und lang andauernde Betarezeptoren-blockierende Wirkungen auf, die anhand von entsprechenden pharmakologischen Versuchen (siehe z.B. Meier et al., Arzneimittelforschung, Bd. 20, S. 1890 (1970) nachgewiesen werden können. So zeigen die neuen Verbindungen eine 50 %-ige Hemmung der Isoproterenol-Tachykardie am isolierten Meerschweinchenherzen (nach Langendorff) in Konzentrationen von etwa 0.001 bis 0.003 µg/ml und eine

50 %-ige Hemmung der Isoproterenol-Tachykardie bzw. -Vasodilatation an der narkotisierten Katze in Dosen von etwa 0.001 bis 0.003 mg/kg bzw. etwa 0.01 bis 0.1 mg/kg bei intravenöser Verabreichung. Die neuen Verbindungen gehören somit zur Klasse der cardioselektiven β-Rezeptorenblocker, d.h. sie hemmen bevorzugt die cardialen Effekte von Isoproterenol gegenüber den vasculären. Die neuen Verbindungen können als Betarezeptoren-Blocker, z.B. bei der Behandlung von Herzrhythmusstörungen (Arrhythmien) und coronaren Herzerkrankungen, wie Angina pectoris, sowie als blutdrucksenkende Mittel in der Behandlung der Hypertonie verwendet werden. Ausserdem besitzen die Verbindungen noch eine cardiostimulierende Wirksamkeit, die sich z.B. am isolierten Vorhof des Meerschweinchens als Zunahme der Herzfrequenz in einem Konzentrationsbereich von etwa 0.01 bis etwa 1 µg/ml nachweisen lässt. Diese Wirkungsqualität lässt sich ferner an der narkotisierten, Resepin- vorbehandelten Katze ebenfalls in Form einer Steigerung der Herzfrequenz in einem Dosisbereich von etwa 0,0003 bis etwa 0,3 mg/kg i.v. nachweisen. Verbindungen mit einer solchen cardiostimulierenden Wirksamkeit beeinträchtigen die Herzfunktion weniger als Stoffe, die diese zusätzlichen Eigenschaften nicht besitzen.

Die Verbindungen zeigen ausserdem Alpharezeptoren-blockierende Eigenschaften, die sich in Konzentrationen von etwa 0,03 bis etwa 0.3 µg/ml z.B. am isoliert-durchströmten Mesenterialbett der Ratte als Hemmung einer Noradrenalin-induzierten Vasokonstriktion oder am isolierten Vas deferens der Ratte als Hemmung der Noradrenalin-induzierten Kontraktion äussern. Ferner weisen die Verbindungen eine längerdauernde blutdrucksenkende Wirkung an der narkotisierten Katze im Dosenbereich von 0,03 bis 3 mg/kg i.v. auf. Durch diese Wirkungskomponenten kann beispielsweise eine Blutdruck-senkende Wirkung begünstigt werden.

Die Erfindung betrifft insbesondere Verbindungen der Formel I worin alk den Aethylenrest bedeutet, und pharmazeutisch annehmbare Säureadditionssalze von solchen Verbindungen.

Die Erfindung betrifft in erster Linie folgende in den Beispielen beschriebenen Verbindungen der Formel I, sowie pharmazeutisch annehmbare Säureadditionssalze dieser Verbindungen:

4-[3-[2-(4-Hydroxyphenoxy)-äthylamino]-2-hydroxypropoxy]-benzimidazol-2-on und

4-[3-[2-(2-Hydroxyphenoxy)-äthylamino]-2-hydroxypropoxy]-benzimidazol-2-on.

Die neuen Verbindungen der vorliegenden Erfindung können in an sich bekannter Weise hergestellt werden.

So kann man die neuen Verbindungen z.B. erhalten, wenn man eine Verbindung der Formel

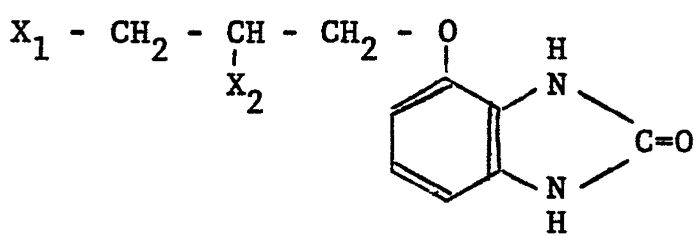

(II)

mit einer Verbindung der Formel

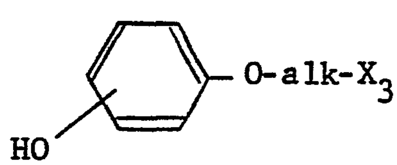

(III)

umsetzt, worin eine der Gruppen $X_1$ und $X_3$ eine reaktionsfähige veresterte Hydroxygruppe darstellt und die andere

für die primäre Aminogruppe steht, und $X_2$ Hydroxy darstellt, oder worin $X_1$ und $X_2$ zusammen die Epoxygruppe bedeuten und $X_3$ für die primäre Aminogruppe steht, und, wenn erwünscht, eine erhaltene freie Verbindung in ein Salz oder ein erhaltenes Salz in eine freie Verbindung überführt, und/oder, wenn erwünscht, ein erhaltenes Racemat in die Antipoden auftrennt.

Eine reaktionsfähige veresterte Hydroxygruppe $X_1$ bzw. $X_3$ ist eine, durch eine starke Säure, insbesondere eine starke anorganische Säure, wie eine Halogenwasserstoffsäure, insbesondere Chlor-, Brom- oder Jodwasserstoffsäure, oder Schwefelsäure, oder eine starke organische Säure, insbesondere eine starke organische Sulfonsäure, wie eine aliphatische oder aromatische Sulfonsäure, z.B. Methansulfonsäure, 4-Methylphenylsulfonsäure oder 4-Bromphenylsulfonsäure, veresterte Hydroxygruppe, und stellt in erster Linie Halogen, z.B. Chlor, Brom oder Jod, oder aliphatisch oder aromatisch substituiertes Sulfonyloxy, z.B. Methylsulfonyloxy oder 4-Methylphenylsulfonyloxy, dar.

Die obige Reaktion wird in an sich bekannter Weise durchgeführt, wobei man, besonders bei Verwendung eines Ausgangsmaterials mit einer reaktionsfähigen veresterten Hydroxygruppe, vorteilhafterweise in Gegenwart eines basischen Mittels, wie einer anorganischen Base, z.B. eines Alkalimetall- oder Erdalkalimetallcarbonats oder -hydroxids, oder eines organischen basischen Mittels, wie eines Alkalimetallniederalkanolats, und/oder eines Ueberschusses des basischen Reaktionsteilnehmers und üblicherweise in Gegenwart eines Lösungsmittels oder Lösungsmittelgemisches und, wenn notwendig, unter Kühlen oder Erwärmen, z.B. in einem Temperaturbereich von etwa -20° bis etwa +150°, in einem offenen oder geschlossenen Gefäss und/oder in einer Inertgasatmosphäre, z.B. in einer Stickstoffatmosphäre, arbeitet.

Die Ausgangsstoffe der Formel II können in an sich bekannter Weise hergestellt werden, z.B. indem man im 4-Hydroxybenzimidazol-2-on oder in einer gegebenenfalls monocyclischen Vorstufe davon die phenolische Hydroxygruppe in die Allyloxygruppe überführt und diese in die gewünschte Gruppe der Formel $X_1-CH_2-CH(X_2)-CH_2-O-$ (IIa) umwandelt. So kann man z.B. in einem 3-Hydroxy-phthalsäure-diniederalkylester die phenolische Hydroxygruppe durch Behandeln mit einem Allylhalogenid, z.B. -bromid, in Gegenwart einer geeigneten Base, wie einem Alkalimetall-, z.B. Kaliumcarbonat, in eine Allyloxygruppe umwandeln, die 3-Allyloxy-phthalsäure aus dem Ester durch Hydrolyse, z.B. durch Behandeln mit einem Alkalimetallhydroxid, freisetzen und, z.B. durch Behandeln mit Essigsäureanhydrid, in das entsprechende Anhydrid überführen. Durch modifizierten Curtius'schen Abbau des so erhältlichen 3-Allyloxy-phthalsäureanhydrids, z.B. durch Behandeln mit einer geeigneten Azidverbindung, wie einem Triniederalkylsilylazid, insbesondere Trimethylsilylazid, erhält man nach anschliessender Hydrolyse das 4-Allyloxy-benzimidazol-2-on. Die Allylgruppe wird z.B. durch Oxidation mit Wasserstoffperoxid oder einer geeigneten anorganischen oder organischen Persäure, z.B. 3-Chlorperbenzoesäure, in die gewünschte 2,3-Epoxy-propylgruppe übergeführt; diese kann man durch Behandeln der entsprechenden Verbindung mit einer geeigneten starken Säure, wie einer Halogenwasserstoffsäure, in eine 2-Hydroxy-3-(reaktionsfähiges-hydroxy)-propylgruppe umwandeln.

Ein weiteres Verfahren zur Herstellung von Verbindungen der Formel I besteht darin, dass man in einer Verbindung der Formel

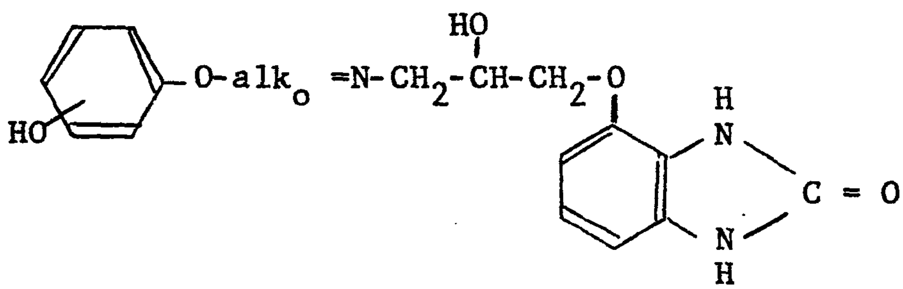

(IV)

wobei $alk_o$ für den einem vicinalen Alkylenrest alk entsprechenden Alkylylidenrest steht, die Gruppierung der Formel $-alk_o = N-$ (IVa) zur Gruppierung der Formel -alk-NH- (IVb) reduziert, und, wenn erwünscht, die zusätzlichen Verfahrensschritte durchführt.

Die obige reduktive Ueberführung eines Restes der Formel IVa in die gewünschte Gruppierung der Formel IVb kann in an sich bekannter Weise durchgeführt werden, wobei man als geeignete Reduktionsmittel insbesondere Leichtmetallhydridreduktionsmittel, wie Alkalimetallborhydride, z.B. Natriumborhydride, sowie Alkalimetallcyanborhydride, z.B. Natriumcyanborhydrid, oder Borhydride, z.B. Diboran, ferner katalytisch aktivierten Wasserstoff, wie z.B. Wasserstoff in Gegenwart eines Schwermetallkatalysators, z.B. Raney-Nickel, Platinoxid oder Palladium, verwenden kann.

Die obigen Reduktionen werden in an sich bekannter Weise, üblicherweise in Gegenwart eines inerten Lösungsmittels, und, wenn notwendig, unter Kühlen oder Erwärmen, z.B. in einem Temperaturbereich von etwa -20° bis etwa +150°, und/oder in einem geschlossenen Gefäss unter Druck und/oder in einer Inertgas-, z.B. Stickstoffatmosphäre, durchgeführt.

Die Ausgangsstoffe der Formel IV kann man in an sich bekannter Weise erhalten, indem man z.B. 4-(2,3-Epoxy-propyloxy)-benzimidazol-2-on mit Ammoniak behandelt und das so erhältliche (3-Amino-2-hydroxy-propyloxy)-benzimidazol-2-on mit einer Carbonylverbindung der Formel

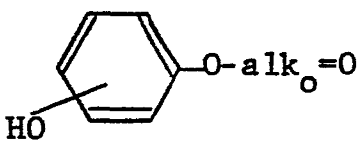

(V)

worin $alk_o$ die oben gegebene Bedeutung hat, umsetzt.

Dabei kann die Herstellung des Ausgangsmaterials der Formel IV gleichzeitig mit dessen Ueberführung in die gewünschte Verbindung der Formel I erfolgen, wenn man die Reaktion der Aminoverbindung mit der Carbonylverbindung der Formel V in Gegenwart eines geeigneten Reduktionsmittels, z.B. in Gegenwart von katalytisch aktiviertem Wasserstoff oder vorzugsweise eines Hydridreduktionsmittels, z.B. Natriumcyanborhydrid vornimmt.

Die neuen Verbindungen der Formel I können ebenfalls erhalten werden, wenn man in einer Verbindung der Formel

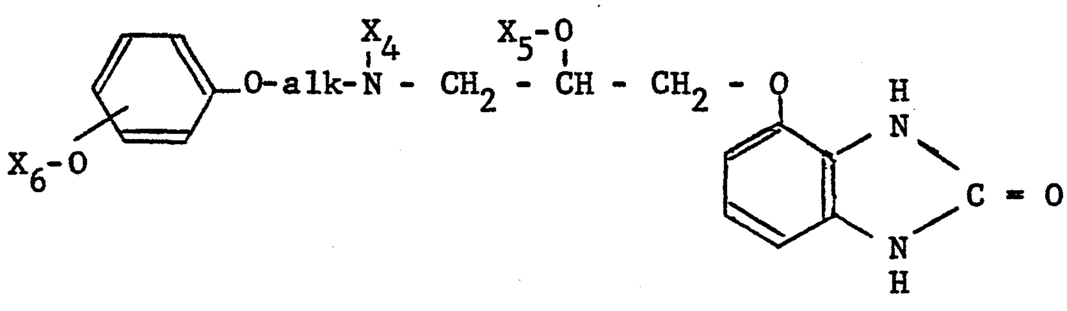

(VI)

worin mindestens eine der Gruppen $X_4$, $X_5$ und $X_6$ eine durch Wasserstoff ersetzbare Gruppe bedeutet und die anderen

für Wasserstoff oder für eine durch Wasserstoff ersetzbare Gruppe stehen, und $X_4$ und $X_5$ zusammen auch einen abspaltbaren, durch zwei, mit dem Sauerstoff- bzw. Stickstoffatom verbundene Wasserstoffatome ersetzbaren Rest darstellen, oder in einem Salz davon die von Wasserstoff verschiedenen der Reste $X_4$, $X_5$ und $X_6$ durch Wasserstoff ersetzt, und, wenn erwünscht, die zusätzlichen Verfahrensschritte durchführt.

Die Abspaltung der Gruppen $X_4$ und/oder $X_5$ und/oder $X_6$ wird mittels Solvolyse oder Reduktion vorgenommen. Dabei ist in den obgenannten Ausgangsstoffen der Formel VI $X_4$ vorzugsweise eine durch Wasserstoff ersetzbare Gruppe, während $X_5$ und $X_6$ in erster Linie für Wasserstoff stehen.

Eine besonders geeignete, abspaltbare Gruppe $X_4$ ist in erster Linie eine hydrogenolytisch abspaltbare α-Arylniederalkylgruppe, wie eine gegebenenfalls substituierte 1-Phenylniederalkylgruppe, worin Substituenten, insbesondere des Phenylteils, z.B. Niederalkyl, wie Methyl oder tert.-Butyl, Hydroxy, Niederalkoxy, wie Methoxy, Halogen, z.B. Chlor oder Brom, und/oder Nitro sein können, und in erster Linie Benzyl. Eine Gruppe $X_4$ kann auch einen solvolytisch, wie hydrolytisch oder acidolytisch, ferner einen reduktiv, inkl. hydrogenolytisch, abspaltbaren Rest, insbesondere einen entsprechenden Acylrest, wie den Acylrest einer organischen Carbonsäure, z.B. Niederalkanoyl, wie Acetyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Iodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, oder den

Acylrest einer organischen Sulfonsäure, wie einer aromatischen Sulfonsäure, in erster Linie einen gegebenenfalls substituierten Phenylsulfonylrest, worin Substituenten z.B. die für den obigen 1-Phenylniederalkylrest gegebene Bedeutung haben, und insbesondere 4-Methylphenylsulfonyl, ferner eine gegebenenfalls substituierte 1-Polyphenyl-niederalkylgruppe, worin Substituenten, in erster Linie des Phenylteils, z.B. die oben gegebene Bedeutung haben, und in erster Linie Trityl darstellen.

Durch Wasserstoff ersetzbare Gruppen $X_5$ und/oder $X_6$ sind vorzugsweise ebenfalls hydrogenolytisch abspaltbare Gruppen, wie eine der obgenannten, gegebenenfalls substituierten 1-Phenyl-niederalkylgruppen und in erster Linie Benzyl. Sie können ferner auch eine der für die Gruppe $X_4$ genannten solvolytisch, inkl. alkoholytisch, oder reduktiv abspaltbaren Acylgruppen, ferner ein, am Verknüpfungskohlenstoffatom polyverzweigter, gegebenenfalls substituierter aliphatischer oder araliphatischer Kohlenwasserstoffrest, wie tert.-Niederalkyl, z.B. tert.-Butyl, oder Trityl sein.

Ein durch $X_4$ und $X_5$ zusammen gebildeter, abspaltbarer Rest ist in erster Linie wiederum eine hydrogenolytisch abspaltbare Gruppe, wie gegebenenfalls substituiertes 1-Phenyl-niederalkyliden, worin Substituenten, z.B. Niederalkyl, wie tert.-Butyl, Hydroxy, Niederalkoxy, Halogen und/oder Nitro sein können, und insbesondere Benzyliden, sowie solvolytisch, insbesondere hydrolytisch spaltbare Gruppen, wie Niederalkyliden, z.B. Methylen oder Isopropyliden, oder Cycloalkyliden, z.B. Cyclohexyliden. Ein weiterer, durch die Gruppen $X_4$ und $X_5$ zusammen gebildeter Rest ist der Diacylrest der Kohlensäure oder Thiokohlensäure, d.h. die Carbonyl- bzw. die Thiocarbonylgruppe.

In der Form von Salzen verwendbare Ausgangsstoffe werden in erster Linie in der Form von Säureadditionssalzen, insbesondere von entsprechenden Salzen mit anorganischen Säuren, z.B. Mineralsäuren, sowie mit organischen Säuren verwendet.

Hydrogenolytisch abspaltbare Reste $X_4$ und/oder $X_5$, und/oder $X_6$, insbesondere gegebenenfalls substituierte 1-Phenyl-niederalkylgruppen, ferner auch geeignete Acylgruppen, wie gegebenenfalls substituiertes 1-Phenylniederalkoxycarbonyl, sowie durch die Gruppen $X_4$ und $X_5$ zusammen gebildete, gegebenenfalls substituierte 1-Phenylniederalkylidengruppen, können durch Behandeln mit katalytisch aktiviertem Wasserstoff, z.B. mit Wasserstoff in Gegenwart eines Nickelkatalysators, wie Raney-Nickel, oder eines geeigneten Edelmetallkatalysators abgespalten werden.

Hydrolytisch abspaltbare Gruppen $X_4$ und/oder $X_5$, und/oder $X_6$, wie Acylreste von organischen Carbonsäuren, z.B. Niederalkanoyl, und Halbestern der Kohlensäure, z.B. Niederalkoxycarbonyl, ferner z.B. Tritylreste, sowie durch die Reste $X_4$ und $X_5$ zusammen gebildete Niederalkyliden-gruppen oder Carbonylgruppe, können je nach Art solcher Reste durch Behandeln mit Wasser unter sauren und/oder basischen Bedingungen, z.B. in Gegenwart einer Mineralsäure, wie Chlorwasserstoff- oder Schwefelsäure, oder eines Alkalimetall- oder Erdalkalimetallhydroxyds oder -carbonats abgespalten werden.

Acidolytisch abspaltbare Reste sind insbesondere gewisse Acylreste von Halbestern der Kohlensäure, wie z.B. tert.-Niederalkoxycarbonyl, oder gegebenenfalls substituierte Diphenylmethoxycarbonylreste, ferner auch tert.- Nieder-alkylreste $X_5$ und/oder $X_6$, sie können durch Behandeln mit

geeigneten starken organischen Carbonsäuren, wie gegebenenfalls durch Halogen, insbesondere Fluor, substituierten Niederalkancarbonsäuren, in erster Linie mit Trifluoressigsäure, wenn notwendig, in Gegenwart eines aktivierenden Mittels, wie Anisol , sowie mit Ameisensäure abgespalten werden.

Unter reduktiv abspaltbaren Resten $X_4$ und/oder $X_5$ und/oder $X_6$ werden auch solche Gruppen verstanden, die beim Behandeln mit einem chemischen Reduktionsmittel, insbesondere mit einem reduzierenden Metall oder einer reduzierenden Metallverbindung, abgespalten werden. Solche Reste sind insbesondere 2-Halogen-niederalkoxycarbonyl oder Aroylmethoxycarbonyl,die z.B. beim Behandeln mit einem reduzierenden Schwermetall, wie Zink, oder mit einem reduzierenden Schwermetallsalz, wie einem Chrom(II)salz, z.B. -chlorid oder -acetat, üblicherweise in Gegenwart einer organischen Carbonsäure, wie Ameisensäure oder Essigsäure, und von Wasser abgespalten werden können. Reduktiv abspaltbare Arylsulfonylreste, besonders diejenigen, die in erster Linie den Rest $X_4$ darstellen, können z.B. beim Behandeln mit einem Alkalimetall, z.B. Lithium oder Natrium, in Ammoniak oder mittels elektrolytischer Reduktion durch Wasserstoff ersetzt werden.

Die obigen Reaktionen werden in an sich bekannter Weise durchgeführt, üblicherweise in Gegenwart eines Lösungsmittels, oder Lösungsmittelgemisches, wobei geeignete Reaktionsteilnehmer gleichzeitig auch als solche funktionieren können, und, wenn notwendig, unter Kühlen oder Erwärmen, z.B. in einem Temperaturbereich von etwa -20° bis etwa +150°, in einem offenen oder geschlossenen Gefäss und/oder in der Atmosphäre eines Inertgases, z.B. Stickstoff.

Die Ausgangsstoffe der Formel VI können in an sich bekannter Weise hergestellt werden, z.B. durch Behandeln einer Verbindung der Formel

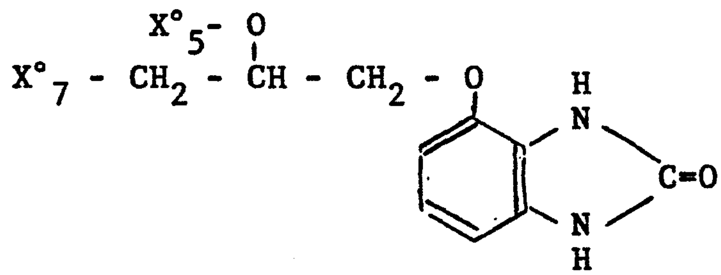

(VII)

mit einer Verbindung der Formel

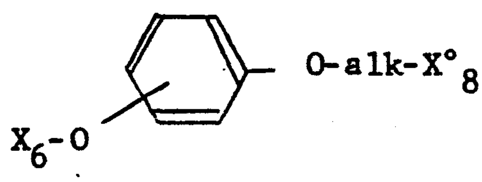

(VIII)

worin $X°_5$ die oben für $X_5$ gegebene Bedeutung hat, und eine der Gruppen $X°_7$ und $X°_8$ eine reaktionsfähige veresterte Hydroxygruppe darstellt, und die andere für die Gruppe der Formel $-NH(X_4)$ steht, worin $X_4$ die oben gegebene Bedeutung hat, mit der Massgabe, dass mindestens eine der Gruppen $X_4$, $X_5$ und $X_6$ von Wasserstoff verschieden ist, oder worin $X°_5$ und $X°_7$ zusammen eine Bindung bilden und $X°_8$ für die Gruppe der Formel $-NH(X_4)$ steht, wobei $X_4$ von Wasserstoff verschieden ist. Die obigen Reaktionen werden in an sich bekannter Weise durchgeführt.

Die neuen Verbindungen der vorliegenden Erfindung können ebenfalls erhalten werden, wenn man aus einer Verbindung der Formel

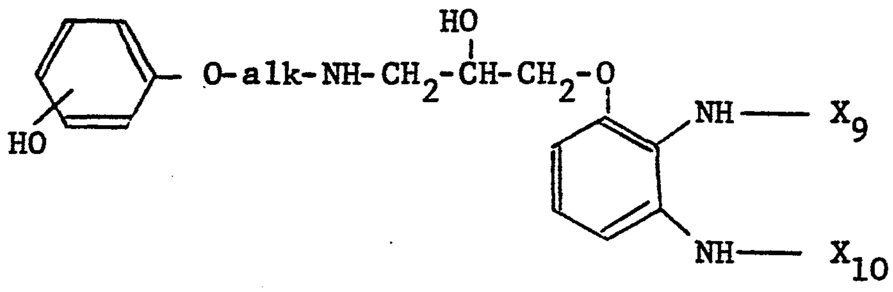

(IX),

worin $X_9$ und $X_{10}$ Reste darstellen, die unter Bildung der in den Verbindungen der Formel I mit den beiden Stickstoffatomen verbundenen Carbonylgruppe abspaltbar sind, oder in einem Salz davon die Reste $X_9$ und $X_{10}$ unter Bildung der Carbonylgruppe abspaltet, und, wenn erwünscht, die zusätzlichen Verfahrensschritte durchführt.

Das Ausgangsmaterial der Formel IX in Form eines Säureadditionssalzes ist z.B. dasjenige mit einer Mineralsäure.

Ueblicherweise stellt einer der Reste $X_9$ und $X_{10}$ Wasserstoff dar, während der andere den Acylrest eines Kohlensäurederivats, wie eines entsprechenden Esters, Halogenids oder Amids bedeutet und z.B. für Niederalkoxycarbonyl, wie Methoxycarbonyl oder Aethoxycarbonyl, oder Halogencarbonyl, wie Chlorcarbonyl oder Aminocarbonyl steht.

Die Reaktion wird in Ab- oder Anwesenheit eines geeigneten Lösungs- oder Verdünnungsmittels, wie eines gegebenenfalls substituierten, z.B. chlorierten, aliphatischen, cycloaliphatischen oder aromatischen Kohlenwasserstoffs, wie Benzol, und, falls notwendig, unter Kühlen oder Erwärmen, z.B. in einem Temperaturbereich von etwa 0° bis etwa 100°, in einem geschlossenen Gefäss und/oder in einer Inertgas-, z.B. Stickstoffatmosphäre durchgeführt. Gegebenenfalls, und vorzugsweise dann, wenn einer der Reste $X_9$ oder $X_{10}$

Aminocarbonyl bedeutet, führt man die Umsetzung in Gegenwart eines basischen Kondensationsmittels, etwa eines Metallalkoholats, wie eines Alkalialkoholats z.B. Natriumäthylat in einem Lösungsmittel, etwa einem Niederalkanol, wie Aethanol durch. Hierbei arbeitet man zweckmässigerweise in einem Temperaturbereich von ca. 0-150°, vorzugsweise von 10 - 120°.

Ausgangsstoffe der Formel IX können in an sich bekannter Weise und gegebenenfalls in situ hergestellt werden. So kann man bevorzugte Ausgangsstoffe erhalten, indem man z.B. im 2,3-Dinitrophenol die phenolische Hydroxygruppe, wie durch Behandeln mit einem Allylhalogenid, z.B. -bromid, in Gegenwart einer Base, z.B. Kaliumcarbonat, in die Allyloxygruppe überführt, diese durch Behandeln des Zwischenproduktes mit Wasserstoffperoxid, z.B. in Gegenwart von Kaliumhydrogencarbonat, oder mit einer geeigneten anorganischen oder organischen Percarbonsäure, z.B. 3-Chlor-perbenzoesäure, in die 2,3-Epoxy-propyloxygruppe und diese, z.B. durch Behandeln des Zwischenprodukts mit einem Amin der Formel

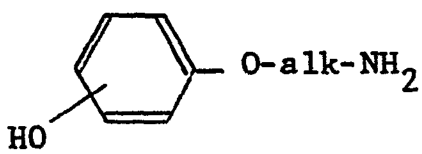

(IIIa)

in die entsprechend substituierte 3-Amino-2-hydroxypropyloxygruppe umwandelt. Darauf werden die beiden Nitrogruppen z.B. durch Behandeln mit katalytisch aktiviertem Wasserstoff, zu den Aminogruppen reduziert. Durch Umsetzen mit einem geeignetn reaktionsfähigen Kohlensäurederivat, z.B. einem entsprechenden Ester, wie einem Diniederalkylcarbonat, z.B. Dimethyl- oder Diäthylcarbonat, einem gemischten Anhydrid, wie Kohlensäuredihalogenid,

z.B. Phosgen, oder einem Amid, z.B. Harnstoff oder N,N'-Carbonyldiimidazol, erhält man ein bevorzugtes Ausgangsmaterial, das vorzugsweise nur in situ gebildet und direkt in eine Verbindung der Formel I umgewandelt wird.

Die neuen Verbindungen der Formel I können ebenfalls erhalten werden, indem man in einer Verbindung der Formel

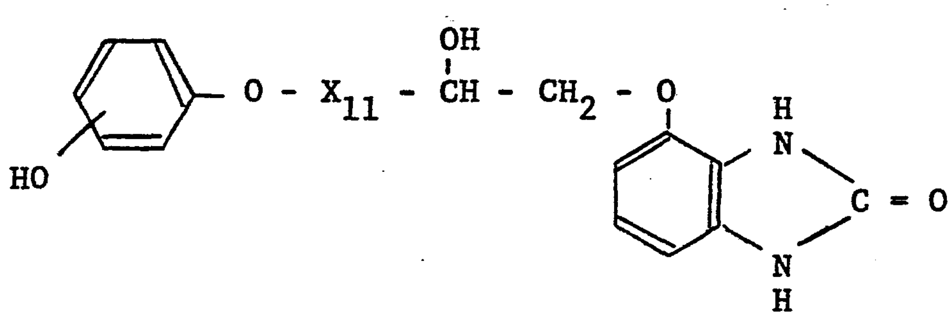

(X)

worin $X_{11}$ einen der Reste der Formeln -alk-NH-(C=$X_{12}$)--(C=$X_{11}$)- (Xa) oder -$alk_a$-(C=$X_{12}$)-NH-$CH_2$- (Xb) darstellt, wobei $alk_a$ eine Methylen- oder Aethylidengruppe und $X_{12}$ den Oxo- oder Thioxorest darstellt, die Gruppe der Formel $X_{11}$ zum Rest der Formel

$$-alk-NH-CH_2- \qquad (XI)$$

reduziert und, wenn erwünscht, die zusätzlichen Verfahrensschritte durchführt. Besonders geeignet zur Reduktion von Gruppen Xa und Xb, die, entsprechend einem Oxorest $X_{12}$, eine Carbamoylgruppierung enthalten, sind Leichtmetallhydridreduktionsmittel, wie Alkalimetallaluminiumhydride, z.B. Lithiumaluminiumhydrid, die sich insbesondere zur Reduktion von Carbamoylgruppen eignen , oder Alkalimetallborhydride, z.B. Natriumcyanborhydrid, oder Borhydride, z.B. Diboran.

Gruppierungen der Formeln (Xa) und (Xb), worin $X_{12}$ jeweils einen Thioxorest bedeutet, werden durch reduktive Entschwefelung, z.B. durch Behandeln mit einem

Hydrierkatalysator, wie Raney-Nickel in die Gruppierung der Formel (XI) umgewandelt.

Die obigen Reduktionsreaktionen werden in an sich bekannter Weise, üblicherweise in Gegenwart eines inerten Lösungsmittels, und, wenn notwendig, unter Kühlen oder Erwärmen, z.B. in einem Temperaturbereich von etwa -20° C bis etwa +150° C, und/oder in einem geschlossenen Gefäss unter Druck und/oder in einer Inertgas-, z.B. Stickstoffatmosphäre, durchgeführt.

Ausgangsstoffe der Formel (X) können durch Umsetzung von Aminoverbindungen der Formel

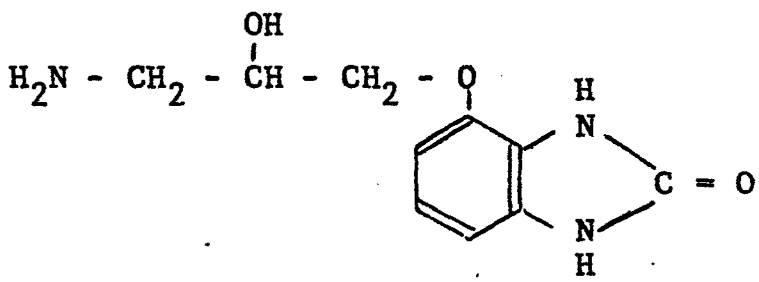

(XII)

worin die Hxdroxygruppe gegebenenfalls in geschützter, z.B. in veresterter oder geeignet verätherter Form vorliegen kann, mit Carbonsäureverbindungen der Formel

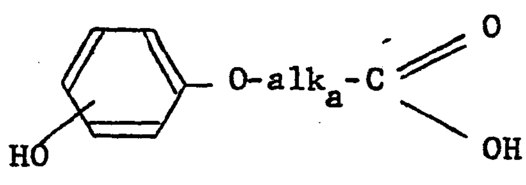

(XIII)

worin $alk_a$ obige Bedeutung hat, oder deren reaktionsfähigen Derivaten, worin die phenolische Hydroxygruppe gegebenenfalls geschützt ist, wie den Halogeniden, z.B. den Chloriden, oder durch Umsetzung von Aminoverbindungen der Formel

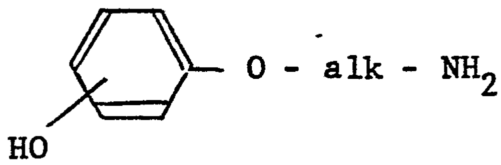

(XIV)

worin die Hydroxygruppe gegebenenfalls geschützt ist, mit der Carbonsäure der Formel

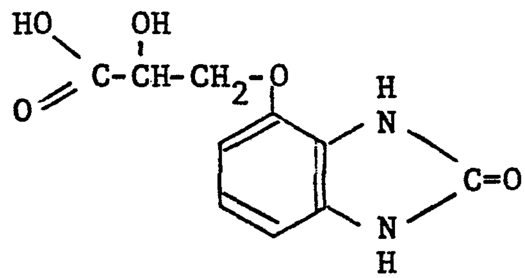

(XV)

worin die Hydroxygruppe gegebenenfalls in geschützter, z.B. in veresterter oder geeignet verätherter Form vorliegen kann, oder deren reaktionsfähigen funktionellen Derivaten erhalten werden. In einem Zwischenprodukt mit einer oder zwei geschützten Hydroxygruppen wird bzw. werden diese in die freie Form übergeführt. Die obigen Reaktionen werden in an sich bekannter Weise durchgeführt.

Je nach den Verfahrensbedingungen und Ausgangsstoffen erhält man die neuen Verbindungen in freier Form oder in der ebenfalls von der Erfindung umfassten Form ihrer Säureadditionssalze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z.B. durch Behandeln mit basischen Mitteln, wie Alkalimetallhydroxiden, -carbonaten oder -hydrogencarbonaten, oder Ionenaustauschern, in die freien Verbindungen übergeführt werden. Andererseits können erhaltene freie Verbindungen in an sich bekannter Weise, z.B. durch

Behandeln mit organischen oder anorganischen Säuren, wie den obgenannten Säuren, Säureadditionssalze bilden, wobei zur Herstellung insbesondere solche Säuren verwendet werden, die sich zur Bildung von pharmazeutisch annehmbaren Salzen eignen.

Diese oder andere Säureadditionssalze der neuen Verbindungen, wie z.B. Pikrate oder Perchlorate, können auch zur Reinigung der erhaltenen freien Basen dienen, indem man die freien Verbindungen in Salze überführt, diese abtrennt und reinigt, und aus den Salzen wiederum die freien Verbindungen bildet.

Die neuen Verbindungen können je nach der Wahl der Ausgangsstoffe und Arbeitsweisen als optische Antipoden oder Racemate vorliegen.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung ein Salz bildenden optisch aktiven Verbindung, insbesondere einer entsprechenden Säure, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchlich als optisch aktive Säuren sind z.B. die D- und L-Formen von Weinsäure, Bis-O,O'-(p-Toluoyl)-weinsäure, Aepfelsäure, Mandelsäure, Camphersulfonsäure, Glutaminsäure, Asparaginsäure oder Chinasäure. Vorteilhafterweise isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden, z.B. wie oben beschrieben, erhalten werden. Neue Ausgangsstoffe bilden ebenfalls einen Gegenstand der Erfindung. Die Erfindung betrifft auch verfahrensgemäss erhältliche Zwischenprodukte.

Die neuen Verbindungen können z.B. in Form pharmazeutischer Präparate Verwendung finden, welche eine pharmakologisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und/oder Schmiermitteln, z.B. Kieselerde, Talk, Stearinsäure oder Salze davon, wie Magnesium- oder Calciumstearat, und/oder Polyäthylenglykol, enthalten. Tabletten können ebenfalls Bindemittel, z.B. Magnesiumaluminiumsilikat,

Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel aufweisen. Ferner kann man die neuen pharmakologisch wirksamen Verbindungen in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffes.

Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 1 mg und etwa 15 mg für Warmblüter mit einem Gewicht von etwa 70 kg.

Die folgenden Beispiele dienen zur Illustration der Erfindung; Temperaturen werden in Celsiusgraden angegeben.

Beispiel 1

Zu einer Suspension von 22,4 g 4-(3-Amino-2-hydroxy-propoxy)-benzimidazol-2-on und 21,2 g Natriumcarbonat in 100 ml Dioxan, welche unter Rückflusskochen unter einer Stickstoffatmosphäre gerührt wird, lässt man im Laufe von 30 Stunden eine Lösung von 22 g 4-Hydroxyphenoxyäthylbromid in 200 ml Dioxan zutropfen. Man lässt 5 Stunden nachreagieren, saugt von den anorganischen Salzen ab, engt das Filtrat unter vermindertem Druck ein, nimmt das zurückgebliebene Oel in Aceton auf und versetzt mit einer ätherischen Chlorwasserstofflösung. Man erhält so das 4-[3-[2-(4-Hydroxyphenoxy)-äthylamino]-2-hydroxy-propoxy]-benzimidazol-2-on-hydrochlorid, welches bei 206 - 208° schmilzt.

Beispiel 2

5,2g 4-[3-{N-Benzyl-N-[2-(4-benzyloxyphenoxy)-äthyl]}-amino]-2-hydroxypropoxy]-benzimidazol-2-on werden in 200 ml Aethanol über 0,5 g 5%-Palladium-auf-Kohle-Katalysator bei 20-25° unter Normaldruck hydriert. Nach Beendigung der Wasserstoffaufnahme wird vom Katalysator abgesaugt, und das Filtrat eingedampft. Aus dem erhaltenen Eindampfrückstand wird nach Lösen in einem Aceton-Methanol-Gemisch durch Zusatz von ätherischer Chlorwasserstofflösung

in üblicher Weise das Hydrochlorid bereitet. Man erhält so das 4-[3-[2-(4-Hydroxyphenoxy)-äthylamino]-2-hydroxypropoxy]-benzimidazol-2-on-hydrochlorid, welches bei 206 - 208° schmilzt.

Der Ausgangsstoff kann wie folgt hergestellt werden:

Ein Gemisch von 3,09 g 4-(2,3-Epoxypropoxy)-benzimidazol-2-on, 4,99 g Benzylaminoäthoxyphenyl-4-benzyläther und 50 ml Isopropanol wird 2 Stunden unter Rühren und Rückfluss zum Sieden erhitzt. Man erhält eine klare Lösung, welche unter vermindertem Druck eingedampft wird. Der Rückstand liefert aus einem Essigsäureäthylester-Aether-Gemisch das 4-[3-(N-Benzyl-N-[2-(4-benzyloxyphenoxy)-äthyl]-amino]-2-hydroxypropoxy]-benzimidazol-2-on, welches bei 108 - 109° schmilzt.

## Beispiel 3

In einer Destillationsapparatur wird eine Suspension von 22,4 g 4-(3-Amino-2-hydroxypropoxy)-benzimidazol-2-on und 20 g 4-Hydroxyphenoxyessigsäuremethylester in 200 ml Xylol unter Rühren zum Sieden erhitzt, wobei darauf geachtet wird, dass die Temperatur im Destillationskopf nicht über 100° steigt. Hierbei destilliert das bei der Reaktion freigesetzte Methanol langsam über. Nach 24 Stunden wird die Temperatur so erhöht, dass das

Xylol überdestilliert. Der Destillationsrückstand, das 4-[3-[2-(4-Hydroxyphenoxy)-acetamido]-2-hydroxypropoxy]-benzimidazol-2-on, wird in absolutem Tetrahydrofuran gelöst und langsam zu einer gerührten Suspension von 7,6 g Lithiumaluminiumhydrid in 200 ml absolutem Tetrahydrofuran getropft. Anschliessend erhitzt man das Reaktionsgemisch noch 5 Stunden unter Rühren und Rückfluss, kühlt anschliessend auf 0° ab und zersetzt den Rückstand durch Zutropfen von 80 ml 12-n. Chlorwasserstoffsäure bei einer Temperatur von 0-5°. Hierauf trägt man 70 g Seignette-Salz ein und stellt das Reaktionsgemisch durch Zusatz von konzentrierter Ammoniak-Lösung auf pH 9-10 ein. Die Reaktionslösung liegt nun als Zweiphasen-Gemisch vor. Die organische Phase wird abgetrennt, über Natriumsulfat getrocknet und unter vermindertem Druck eingedampft. Das zurückbleibende, rohe 4-[3-[2-(4-Hydroxy-phenoxy)-äthylamino]-2-hydroxypropoxy]-benzimidazol-2-on liefert mit Salzsäure das Hydrochlorid, welches nach dem Umkristallisieren aus Aceton bei 206-208° schmilzt.

Das als Ausgangsmaterial verwendete 4-(3-Amino-2-hydroxypropoxy)-benzimidazol-2-on kann wie folgt hergestellt werden:

a) 3,3 g 4-(2,3-Epoxypropoxy)-benzimidazol-2-on und 11,8 g Benzylamin werden in 80 ml Isopropanol 5 Stunden unter Rückfluss zum Sieden erhitzt. Dann wird unter vermindertem Druck das Lösungsmittel samt dem überschüssigen Benzylamin abdestilliert und das zurückgebliebene Oel bei 90°/0,001 Torr 2 Stunden getrocknet. Es besteht aus 4-(3-Benzylamino-2-hydroxy-propoxy)-benzimidazol-2-on, welches aus Wasser in Kristallen erhalten wird, die nach dem Trocknen bei 60°/1 Torr während 18 Stunden bei 203-205° schmelzen.

Das Hydrochlorid schmilzt bei 231-232° unter Zersetzung.

b) Eine Lösung von 15,5 g 4-(3-Benzylamino-2-hydroxy-propoxy)-benzimidazol-2-on in 160 ml Methanol wird mit 1,6 g 5%-Palladium-auf-Kohle-Katalysator versetzt und bis zur Aufnahme der berechneten Wasserstoffmenge hydriert. Nach Filtration vom Katalysator und Abdestillieren des Lösungsmittels erhält man das 4-(3-Amino-2-hydroxy-propoxy)-benzimidazol-2-on, welches nach dem Umkristallisieren aus Aethanol bei 188-190° schmilzt.

Das Hydrochlorid schmilzt bei 232-239°.

Beispiel 4

9.5 g 4-[3-[N-Benzyl-N-[2-(2-benzyloxyphenoxy)-äthyl]-amino]-2-hydroxypropoxy]-benzimidazol-2-on werden unter Zusatz von 18 ml 1-n. methanolischer Chlorwasserstoffsäure in 100 ml Methanol gelöst und anschliessend über 1,0 g 5 %-Palladium-auf-Kohle-Katalysator bei 20-25° unter Normaldruck hydriert. Nach Beendigung der Wasserstoffaufnahme verdünnt man mit Methanol-Wasser-Gemisch (1:1, v/v), bis das ausgefallene Produkt gelöst ist. Hierauf filtriert man vom Katalysator ab und dampft das Filtrat ein. Aus dem erhaltenen Eindampfrückstand erhält man nach Umkristallisation aus heissem Wasser das 4-[3-[2-(2-Hydroxyphenoxy)-äthylamino]-2-hydroxypropoxy]-benzimidazol-2-on-hydrochlorid, welches bei 256-257° schmilzt.

Der Ausgangsstoff kann wie folgt hergestellt werden:

Ein Gemisch von 6,86 g 4-[2,3-Epoxypropoxy)-benzimidazol-2-on, 11,1 g Benzylaminoäthoxyphenyl-2-benzyläther und 150 ml Isopropanol wird 3 Stunden unter Rühren und Rückfluss zum Sieden erhitzt. Dann wird das Lösungsmittel unter vermindertem Druck abdestilliert und der Rückstand in heissem Methanol aufgenommen. Beim Abkühlen kristallisiert das 4-[3-[N-Benzyl-N-(2-benzyloxyphenoxy)-äthyl]-amino]-2-hydroxypropoxy]-benzimidazol-2-on, welches bei 152-153° schmilzt.

**Beispiel 5**

Tabletten enthaltend 0,002 g 4-[3-[2-(4-Hydroxyphenoxy)-äthylamino]-2-hydroxy-propoxy]-benzimidazol-2-on-hydrochlorid, werden wie folgt hergestellt:

Zusammensetzung (für 1000 Tabletten):

| | |
|---|---|
| 4-[3-[2-(4-Hydroxyphenoxy)-äthylamino]-2-hydroxy-propoxy)-benzimidazol-2-on-hydrochlorid | 2,00 g |
| Milchzucker | 35,00 g |
| Maisstärke | 50,00 g |
| Kolloidale Kieselsäure | 6,00 g |
| Talk | 6,00 g |
| Magnesiumstearat | 1,00 g |
| Wasser q.s. | |

Das 4-[3-[2-(4-Hydroxyphenoxy)-äthylamino]-2-hydroxy-propoxy]-benzimidazol-2-on-hydrochlorid wird mit dem Milchzucker, einem Teil der Maisstärke und mit kolloidaler Kieselsäure gemischt und die Mischung durch ein Sieb getrieben. Ein weiterer Teil der Maisstärke wird mit der fünffachen Menge Wasser auf dem Wasserbad verkleistert und die Pulvermischung mit diesem Kleister angeknetet, bis eine schwach plastische Masse entsteht. Diese wird durch ein Sieb von etwa 3 mm Maschenweite gedrückt, getrocknet und das trockene Granulat nochmals durch ein Sieb getrieben. Darauf werden die restliche Maisstärke, der Talk und das Magnesiumstearat zugemischt und die erhaltene Mischung zu Tabletten von 0,100 g Gewicht (mit Bruchkerbe) verpresst.

In analoger Weise können andere Verbindungen der Formel I oder deren pharmazeutisch annehmbare, nicht-toxischen Säureadditionssalze, z.B. das 4-[3-[2-(2-Hydroxyphenoxy)-äthylamino]-2-hydroxy-propoxy]-benzimidazol-2-on-hydrochlorid, als Wirkstoffe in den beschriebenen Tabletten verwendet werden.

Patentansprüche

1. Verätherte Hydroxybenzodiheterocyclen der Formel

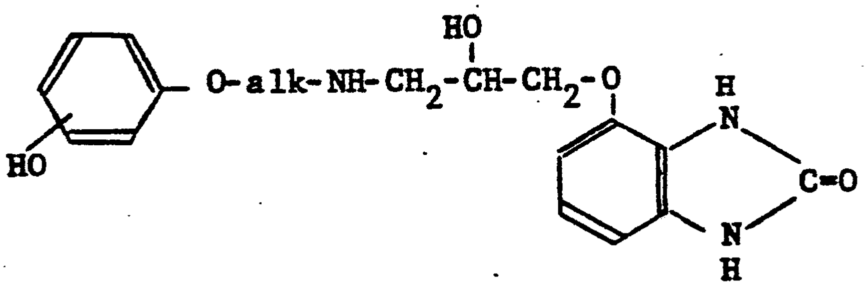

(I)

worin alk vicinales Alkylen mit 2 - 3 Kohlenstoffatomen bedeutet.

2. Verbindungen der Formel I gemäss Anspruch 1, worin alk den Aethylenrest bedeutet.

3. 4-[3-[2-(4-Hydroxyphenoxy)-äthylamino]-2-hydroxypropoxy]-benzimidazol-2-on.

4. 4-[3-[2-(2-Hydroxyphenoxy)-äthylamino]-2-hydroxypropoxy]-benzimidazol-2-on.

5. Säureadditionssalze der Verbindungen gemäss einem der Ansprüche 1 bis 4.

6. Pharmazeutisch annehmbare, nicht-toxische Säureadditionssalze der Verbindungen gemäss einem der Ansprüche 1 bis 4.

7. Pharmazeutische Präparate enthaltend eine Verbindung gemäss einem der Ansprüche 1 bis 4 und 6.

8. Eine Verbindung gemäss einem der Ansprüche 1 bis 4 und 6 zur Verwendung als Betarezeptoren-blockierende Mittel, insbesondere zur Behandlung von Herzrythmusstörungen und coronaren Herzerkrankungen, sowie als blutdrucksenkende Mittel in der Behandlung der Hypertonie.

9. Verfahren zur Herstellung von verätherten Hydroxybenzodiheterocyclen der im Anspruch 1 angegebenen Formel I, in welcher alk die dort definierte Bedeutung hat, und ihrer Säureadditionssalze, dadurch gekennzeichnet, dass man

a) eine Verbindung der Formel

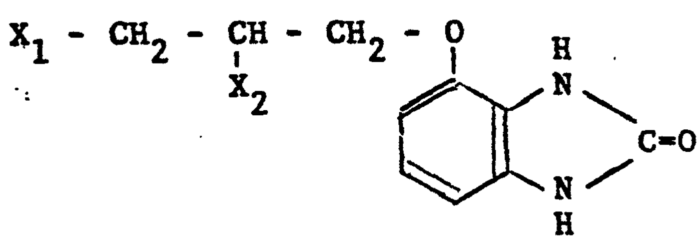

(II)

mit einer Verbindung der Formel

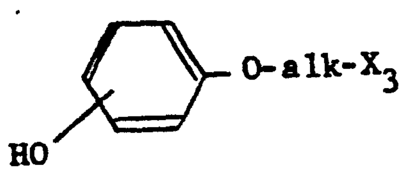

(III)

umsetzt, worin eine der Gruppen $X_1$ und $X_3$ eine reaktionsfähige veresterte Hydroxygruppe darstellt und die andere für die primäre Aminogruppe steht, und $X_2$ Hydroxy darstellt, oder worin $X_1$ und $X_2$ zusammen die Epoxygruppe bedeuten und $X_3$ für die primäre Aminogruppe steht, oder

b) in einer Verbindung der Formel

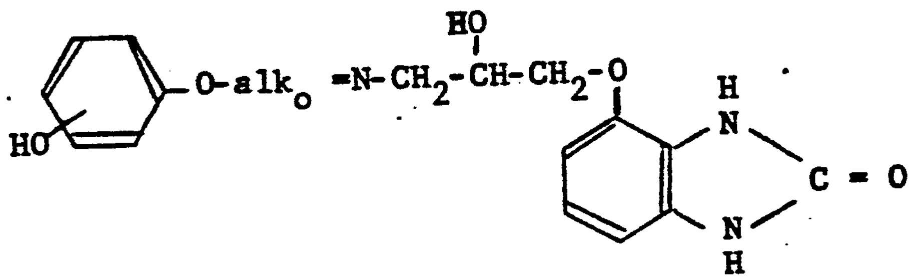

(IV)

wobei $alk_o$ für den einem vicinalen Alkylenrest alk entsprechenden Alkylylidenrest steht, die Gruppierung der Formel $-alk_o = N -$ (IVa) zur Gruppierung der Formel $-alk-NH-$ (IVb) reduziert, oder

c) in einer Verbindung der Formel

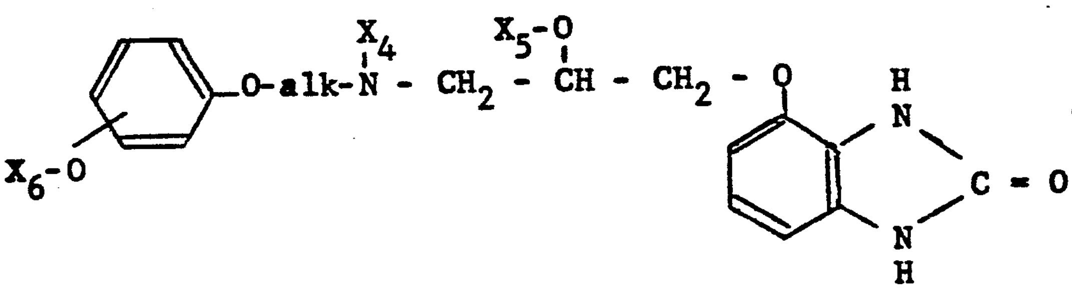

(VI)

worin mindestens eine der Gruppen $X_4$, $X_5$ und $X_6$ eine durch Wasserstoff ersetzbare Gruppe bedeutet und die anderen für Wasserstoff oder für eine durch Wasserstoff ersetzbare Gruppe stehen, und $X_4$ und $X_5$ zusammen auch einen abspaltbaren, durch zwei, mit dem Sauerstoff- bzw. Stickstoffatom verbundene Wasserstoffatome ersetzbaren Rest darstellen,

oder in einem Salz davon die von Wasserstoff verschiedenen der Reste $X_4$, $X_5$ und $X_6$ durch Wasserstoff ersetzt, oder

d) in einer Verbindung der Formel

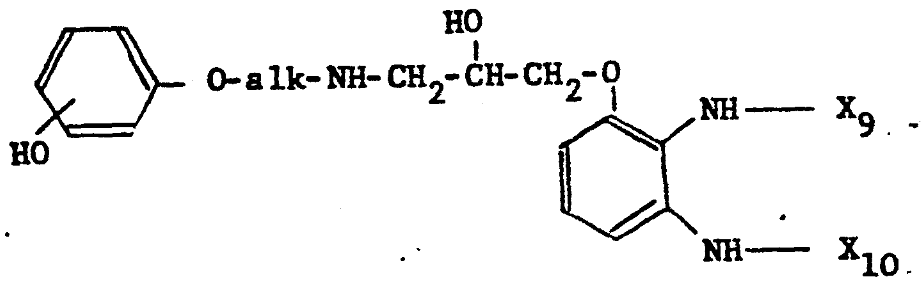

(IX),

worin $X_9$ und $X_{10}$ Reste darstellen, die unter Bildung der in den Verbindungen der Formel I mit den beiden Stickstoffatomen verbundenen Carbonylgruppe abspaltbar sind, oder in einem Salz davon die Reste $X_9$ und $X_{10}$ unter Bildung der Carbonylgruppe abspaltet, oder

e) in einer Verbindung der Formel

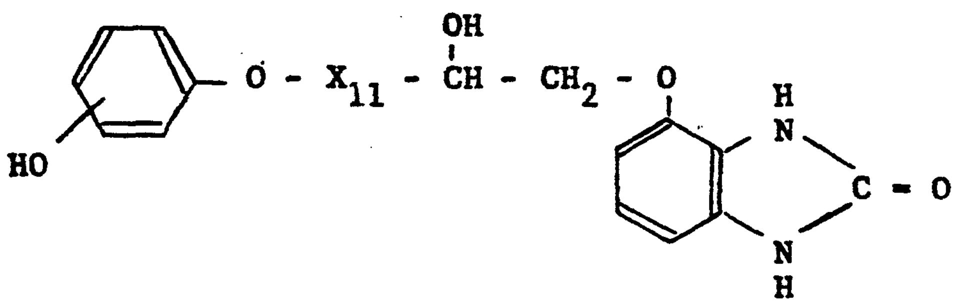

(X)

worin $X_{11}$ einen der Reste der Formeln $-alk-NH-(C=X_{12})-$ $-(C=X_{11})-$ (Xa) oder $-alk_a-(C=X_{12})-NH-CH_2-$ (Xb) darstellt, wobei $alk_a$ eine Methylen- oder Aethylidengruppe und $X_{12}$ den Oxo- oder Thioxorest darstellt, die Gruppe der Formel $X_{11}$ zum Rest der Formel

$$-alk-NH-CH_2- \qquad (XI)$$

reduziert, und, wenn erwünscht, eine erhaltene freie Verbindung in ein Salz oder ein erhaltenes Salz in eine freie Verbindung überführt, und/oder, wenn erwünscht, ein erhaltenes Racemat in die Antipoden auftrennt.

0003758

Europäisches Patentamt

# EUROPÄISCHER RECHERCHENBERICHT

Nummer der Anmeldung
EP 79 10 0273

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| A | DE - A - 2 700 193 (CIBA-GEIGY)<br>* Seiten 12-15 *<br>& BE - A - 850 166<br>& FR - A - 2 337 718<br>& NL - A - 77 00141<br>-- | 1 | C 07 D 235/26<br>A 61 K 31/415 |
| A | FR - A - 1 583 153 (I.C.I.)<br>* Seiten 1,2 *<br>& BE - A - 651 032<br>& CH - A - 468 372<br>& CH - A - 472 402<br>& CH - A - 473 800<br>& CH - A - 476 719<br>& CH - A - 486 442<br>& DE - A - 1 493 853<br>& NL - A - 64 08650<br>& GB - A - 1 058 822<br>& FR - M 4161<br>-- | 1 | RECHERCHIERTE SACHGEBIETE (Int. Cl.²)<br>C 07 D 235/26 |
| P | DE - A - 2 819 458 (HOFFMANN LA ROCHE)<br>* Seiten 12,13,16,17,28,29 *<br>& BE - A - 866 596<br>& FR - A - 2 399 414<br>& NL - A - 78 04794<br>---- | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Den Haag | 15-05-1979 | DE BUYSER |